# EUROPEAN PATENT SPECIFICATION

(11) **EP 4 604 902 B1**
(45) Date of publication and mention of the grant of the patent: **01.07.2026**
(21) Application number: 23809345.4
(22) Date of filing: 20.10.2023
(51) Int. Cl.: A61H 5/00

(54) **SYSTEM FOR DICHOPTIC TREATMENT OF AMBLYOPIA USING BOTH VISUAL AND AUDITORY STIMULI**
SYSTEM ZUR DICHOPTISCHEN BEHANDLUNG VON AMBLYOPIE UNTER VERWENDUNG VON VISUELLEN UND AUDITIVEN STIMULI
SYSTÈME DE TRAITEMENT DICHOPTIQUE DE L'AMBLYOPIE À L'AIDE À LA FOIS DE STIMULI VISUELS ET AUDITIFS

(30) Priority: 20.10.2022 NL 2033360
(43) Date of publication of application: 27.08.2025
(73) Proprietor: Vedea Healthware B.V., 1087 JA Amsterdam (NL)
(72) Inventor: JANSEN, Daniel Johannus, 1087 JA Amsterdam (NL); AALBERS, Teun, 1087 JA Amsterdam (NL); WIJNGAARDE, Joël, 1087 JA Amsterdam (NL)
(74) Representative: Leeger, Ferdinand Christiaan
(86) International application number: PCT/NL2023/050555
(87) International publication number: WO 2024/085762

(56) References cited:
- EP-A1- 3 420 889
- CN-A- 102 813 500
- US-A1- 2019 290 528

## Description

The present invention relates to a system for the treatment of Amblyopia, in particular in children.

Amblyopia is characterized by poor or indistinct vision in an eye. It is one of the most common ophthalmic disorders in children; it affects 1-5% of the population, and is generally associated with a history of strabismus, anisometropia, or deprivation of vision early in life by vision-obstructing disorders such as congenital cataract. Amblyopia is a developmental problem in the brain, not an organic problem in the eye. The part of the brain receiving the visual information from an eye is not stimulated properly, and develops abnormally. This abnormal development negatively affects a person's visual perception through said eye. An eye for which the visual perception is affected in the brain is considered a weaker eye, also known as an Amblyopic eye (AE).

The problem of Amblyopia is typically addressed in the following manner: First, the underlying condition that initially disadvantaged the weaker eye (i.e., the need for glasses, obstruction by a ptotic eyelid, strabismus, etc) is corrected. Second, the better-seeing eye is temporarily disadvantaged relative to the amblyopic eye. This is usually accomplished via occlusion of the dominant eye with an eye patch or similar occlusive device. In lieu of a patch, it is also possible to use cycloplegic eye drops such as atropine in the dominant eye to cause visual blurring. This practice is sometimes referred to as "pharmacologic patching". In this way, the amblyopic eye is given an opportunity to form sufficient synaptic connections to allow useful vision before the critical period of development is complete and synaptic connections become fixed. If left undiscovered and untreated an adult may never fully recover the use of his amblyopic eye.

Still, some plasticity remains in the brain, and partial recovery of function is possible through methods, such as known from EP3329838 B1 by the Canadian McGill University, which subject adults to a binocular vision assessment and therapy in which the information content between eyes is adjusted gradually based on the change in continuously assessed binocular vision. Each of the known methods is exclusively focused on treating Amblyopia as a unisensory visual impairment.

Other prior art is also known from:
EP 3 420 889 A1 describes an apparatus for checking the eyesight of a user has a first lens that can be selectively closed to prevent light from reaching a first eye of the user (202) and a second lens (103) for displaying an image for viewing by a second eye of the user.

US 2019/290528 A1 concerns a method for treating amblyopia in a patient includes: providing a virtual reality headset having a display that provides a first display to a left eye and a second display to the right eye; and manipulating one of the first display or the second display such that the manipulation encourages a patent's amblyopic eye to perform more work than the non-amblyopic eye.

CN 102813500 relates to a perception correcting and training system on the basis of the binocular integration which comprises an integration perception function checker, an image controller controlled by a computer, an image branching device, 3D (three-dimensional) display equipment, a signal transmitter, 3D spectacles, a signal receiver of the 3D spectacles and a solid figure picture database.

However, the present invention is the first of its kind that provides a more effective treatment by focusing on the broader cross-modal interaction in spatial temporal perception. More specifically, applicants' approach is unique in that it sets out to reduce the suppressive influences exerted by the fellow eye on the amblyopic eye through the use of sound.

### SUMMARY OF THE INVENTION

The present invention is directed to a system according to claim 1,17 and a computer-implemented method according to claim 15.

Additional features and embodiments of the invention are defined in the dependent claims.

### SUMMARY OF THE DISCLOSURE

According to a first aspect of the invention there is thus provided:
A system for implementing a method of treatment of amblyopia, comprising:
- a computer programmed to generate a virtual environment with first user associated task reflective of binocular perception, wherein the computer is further designed to produce a left eye image stream and right eye image stream of said environment adapted to be viewed dichoptically as a pair and perceived with binocular vision;
- a dichoptic display presenting a selected one of said image streams as a right eye image stream to a first user's right eye and a left eye image stream to a first user's left eye; and
- a sound system, such as headphones or a 360 degree surround system, comprising a plurality of sound sources,
wherein the computer is programmed to manifest within said virtual environment at least one visual object in a first direction relative to the first user, and wherein the computer is programmed to produce sound streams, also known as audio streams, of said environment, namely a continuous 3D soundscape comprising both environmental sounds and sounds pertaining to a manifested object, via the sound system for directing the visual attention of the first user in said first direction within the virtual environment such that, in use, auditory perception of the visual object by the first user precedes or coincides, such as exclusively precedes or coincides, with a visual perception of the visual object by the first user, and wherein the visual streams have a mutual difference in information content, such as a different signal to noise ratio, between said left eye image stream and said right eye image stream. The person skilled in the art will understand that there are many ways to cause the auditory perception of a visual object to precede or coincide with the visual perception of said visual object. The object may be programmed to visually appear after a predetermined period of time, such as 1-1000 milliseconds, preferably at least exceeding a refresh time of the display >1/60^{th} of a second, once the sound pertaining to the object is generated, or by manifesting the object beside or behind the user outside of the users' view. The sound streams may optionally comprise a left ear sound stream and a right ear sound stream, such as in case the sound system is a pair of headphones, and wherein the streams comprise frequency, timing and/or loudness differences. This beneficially enables an accelerated visual perception of the object in the first user, and provides said user with an immersive 3D sound experience that enables an auditory stimulus to be mentally placed in a particular direction. The sound streams may comprise at least one auditory stimulus event.

The skilled person will fully understand that left and right eye image streams can be viewed dichoptically by producing image streams corresponding to each of the eyes being spaced apart. This also allows dichoptic vision within a VR environment to be three dimensional. Optionally, the system may be designed so receive an input pertaining to the specific distance between the user's eyes, or portions of the user's eyes, and to adjust a viewpoint distance between the image streams based on said input.

A soundscape can be considered a combination of sounds that forms an immersive environment, or more precisely an acoustic immersive environment. An acoustic immersive environment here being an artificial interactive computer-created acoustic environment that forms part of the virtual environment on which basis the visual streams are produced. In this environment several environmental elements, such as a tree swaying in the wind, a stream of water, falling droplets and more elements, have sounds, or can solicit sound through user interaction, corresponding to a direction and distance from the user. It is within such an environment that the user is allowed to isolate a sound corresponding to a manifested object or event. Surprisingly, the immersive acoustic environment allows a user to improve his or her overall situational awareness as the manifested object or event can be perceived in relation to environmental elements. In that sense environmental noises are different from background noises that merely effect signal-to-noise ratio of the sounds associated with the manifested object or event. It was found that this enhances visual performance, and accelerates improvement in visual perception.

In some situations, the introduction of a background noise, or additional background noise, in combination with environmental acoustics can be used to increase the difficulty of a gaming environment, and can prove effective as a step in a method of treating amblyopia as the user's visual capabilities improve, or as the user progresses through different virtual environments.

Optionally, the system is designed, such as by having the computer programmed, to repeat a task at least once wherein the mutual difference in information content, such as signal to noise ratio, is preserved, but switched between the image streams. This can for example mean that the signal to noise ratio of the left eye image stream becomes the signal to noise ration of the right eye image stream and vice versa. By repetition of the task 'information' about the performance of said task, such as successful completion, time for completion, and/or degree of completion, can be used to determine which eye is the weaker eye. The information reflective of the poorer or poorest, performance may be used by the device to determine the image streams by their respective mutual difference are selected correctly for treatment or non-medical perceptual training. Beneficially, this allows the system to automatically establish which eye is the weaker eye and does not require another user, or the user him or herself, to indicate to the system which is the weaker eye that needs to be trained. A method may also use any one of these steps for automatically establishing the weaker eye. Alternatively, this can be manually selected.

Further optionally, the system may be designed, such as by the computer being programmed, to maintain a higher signal to noise ratio in the image stream to the weaker eye, compared to that of the stronger eye.

The system is designed to assume amblyopia in its user, it specifically precludes any step of testing whether the user actually suffers from amblyopia. The system may, such as by its computer programming, omit checking whether information pertaining to the effective performance of the task reflective of binocular perception meets a predefined standard. The device simply provides an environment in which amblyopia is treated if present.

Additionally to the above, and compatible with all embodiments, the computer is programmed to produce the sound streams such that these have a mutual difference in information content, such as a different signal to noise ratio, between the left ear sound stream and the right ear sound stream. Simply put, the above option further improves the efficacy of visual localization treatment in Amblyopic users by incorporating audio treatment steps analogues to the visual treatment steps.

Optionally, the sound can be provided such that, when in use, the signal to noise ratio of the left ear is reduced with respect to the right ear, when in use, the object is present in a first user's left hemifield of vision, and wherein the signal to noise ratio of the right ear is reduced with respect to the left ear, when in use, the object is present in a first user's right hemifield of vision, or vice versa.

It is noted that the system does not require eye tracking to accomplish the above. Without eye tracking the above method assumes that the object when displayed on the left half of a screen portion provided for the left eye, and a left half of a screen portion provided for the right eye is in the left hemifield of the first user's vision; whereas the object when displayed on the right half of a screen portion provided for the left eye, and a right half of a screen portion provided for the right eye is in the right hemifield of the first user's vision.

As a matter of definition it is noted that the right half of the brain has visual pathways for the left hemifield of both eyes, and the left half of the brain has visual pathways for the right hemifield of both eyes. More simply put, the right and left visual field is the visual field of an eye on the left and right side of a vertical midline of said eye respectively.

Eye tracking can also be omitted if treatment methods are based on a measured performance related to tasks associated with a manifested object. In other words, the measured performance comprises indirect information on the position of the eye. It is safe to assume that an object is not perceived correctly when the user fails to interact with the object, fails to complete a task associated with handling the virtual object, or if the amount of time associated with completing the task exceeds a predetermined threshold for example. A system implementing a treatment method based on measured performance may simply be designed without eye tracking devices and/or be programmed such that the method makes no use of eye tracking. This reduced the computational requirement of the treatment method, which allows the system to have lower system specifications and a lower energy consumption.

Alternatively, the sound can be provided such that the signal to noise ratio of only one ear, such as a weaker ear, of the left and right ear is increased with respect to the other. This method is exceedingly beneficial for amblyopic patients having a distinct weaker ear.

It is also separately from the above possible for the audio streams to comprise at least one sound unrelated to the direction of the object, such as an environmental sound, and at least one sound related to the direction of the object. In combination with the before mentioned option the loudness of the sound unrelated to the direction of the object may be considered the noise, and the loudness of the sound related to the direction of the object may be considered the signal, in the signal to noise ratio.

This is intended to prompt a user to focus in a direction in which the binocular images comprise a visual object or event for being perceived by the user. A source for the images could be a dichoptic screen, and a source for sound could be a pair of headphones or a 360 degree surround system. A dichoptic screen can be a normal screen that is set up so that visual information is presented dichoptically. In one example, the screen can be split between a left and right eye portion, and optionally a shielding portion may be provided so that left eye images and right eye images are separated. By information content applicants mean the overall luminance, local contrast, motion direction, motion speed, spatial sampling, spatial frequency and orientation of local image features. While the present approach uses a signal/noise paradigm it could be generalized to other stimuli where the information content of left and right images are systematically varied in a way that lends itself to a quantitative measure of the extent to which information from the two eyes are combined binocularly.

In a more detailed example, the system is provided for the treatment of amblyopia, comprising: - a computer programmed to generate a virtual environment, such as a 2D or 3D virtual environment, with first user associated task reflective of binocular perception, wherein the computer is further designed to produce a left eye image stream and right eye image stream of said environment adapted to be viewed dichoptically as a pair and perceived with binocular vision, said streams having a difference in information content, such as a different signal to noise ratio, between said left eye image stream and said right eye image stream;
- a dichoptic display presenting a selected one of said image streams as a right eye image stream to a first user's right eye and a left eye image stream to a first user's left eye;
wherein the system further comprises: a sound system designed for providing sounds to a left ear and to a right ear of the first user, wherein the sounds provided to the left ear and right ear are mutually different, such as in volume or in timing, for drawing the first user's visual attention to a location within the virtual environment. Difference in volume and timing can give a sense of directionality to the sound. This allows the user to become spatially aware of a sound producing event allowing this to guide the user's visual perception.

In practice, the user is provided with a virtual environment in which he or she is expected to perform tasks, such as retrieving, catching or dodging an object or retrieving information from the virtual environment to solve a problem or puzzle. For the completion of tasks time may be limited so that the degree of Amblyopia may correspond to a certain level, which cannot be completed without dichoptic improvement. A sound may be hearing the bouncing of a ball, the task may be to catch the ball. To catch the ball, such as in between bounces, the first user must visually perceive the ball. The person skilled in the art will know that there are a plethora of possible sounds one could associate with any number of tasks. The only thing that matters is that the user's attention is visually drawn to the perceived direction of the sound. This is sufficient to allow the user to establish a degree of spatial awareness.

For improvement, the system may present the first user with various different environments, each with a different degree of information content for the left and right image streams. That is to say, preferably, the difference in information content is variable between said left eye image stream and said right eye image stream. To improve user experience a sense of control over the increase in difficulty is provided. To this end the system may comprise a controller for allowing a first user's input to perform the associated task, wherein the computer is programmed to change the variable difference in information content between said left eye image stream and said right eye image stream in response to the first user's performance. The controller may be a hand held controller that can be connected to the computer, such as wirelessly. In one example the Bluetooth or Wifi connectable.

In a more specific embodiment the system comprises a virtual reality head mount to be worn by the first user. Here the computer can be designed as a smartphone assembled with said head mount. The dichoptic display would in this example be the screen comprised in the smartphone. For such a screen to function as a dichoptic display the images for left and right eye may be displayed on mutually different halves of the same screen, wherein the phone is assembled with the head mount such as to present each half to a mutually different eye of the first user. Children are known to have a relatively short attention span. This prevents extended periods of focus. Binaural sound, or directional sound, improves this focus. Furthermore, a diversity of training content also increases the effectiveness of the treatment. Therefore, the system may comprise a distant or local server provided with a digital library of programs for generating mutually different environments. The computer would in this case be designed to retrieve the virtual environment and associated task from said server. The environment itself could be changed in response to the first user's performance. Additionally or alternatively, a second human interface may be comprised within the system, which allows a second user to change the environment by selecting a program from the library.

The system may be designed to record task performance information of the first user. The computer or distant server may store the performance information in an associated memory. Performance information may take the form of records of task completion, speed of completion and moment of completion. The system may then comprise a separate human interface for a second user, wherein the interface is designed to allow the second user to access task performance information of the first user via said separate human interface. This allows the treating specialist to monitor the improvement of the first user over time.

Optionally, the sound system is designed as a 360 degree sound system, which enables the sounds to come from all directions with respect to the first user. This beneficially allows audio perception and thus spatial temporal placement of an object or event in the virtual environment to precede the visual perception of the object or event. This reinforces cross-sensory perception. This needn't always be the case, but an object or event to be perceived and tied to a task may manifest somewhere in the virtual environment behind the first user, beside the first user, or only in the field of vision of one eye of the first user. That is to say, the system can be programmed to manifest an object or event such that it is not shown, or not shown to both eyes, until the user moves his or her head towards the direction of the sound. Alternatively, the sound system is designed as a headset, earbuds, or headphones presenting sounds binaurally.

As the user may move his head during the treatment, this may mean that sound may not always be perceived as coming from the intended direction with respect to the user. As such, the system may comprise an orientation sensor, such as a 3D G-sensor or 3D magnetic field sensor. These are sensors common to smart phones. The computer is preferably a smartphone, and may in this case use its orientation sensor to determine a change in direction of the first user's head using said sensor. The computer may be designed to adjust the direction of the sounds in response to the determined change in direction of the first user's head. This makes it easier for children in particular.

According to a second aspect of the invention there is provided a computer-implemented method comprising the step of:
- generating a virtual environment with first user associated task reflective of binocular perception;
- producing a left eye image stream and right eye image stream of said environment adapted to be viewed dichoptically as a pair and perceived with binocular vision;
- presenting a selected one of said image streams as a right eye image stream to a first user's right eye and a left eye image stream to a first user's left eye; and
- providing sounds to a left ear and sounds to a right ear of the first user, wherein the sounds provided to the left ear and right ear are mutually different, such as in volume, for attracting the first user's visual attentions towards a task within the virtual environment.

Optionally, the computer-implemented method comprising the further steps of:
- changing the variable difference in information content between said left eye image stream and said right eye image stream in response to the first user's performance; and
- receiving a first user's input in the performance of the associated task.

The invention will be better understood by way of the following detailed description of a preferred embodiment, with reference to the appended drawing, in which:

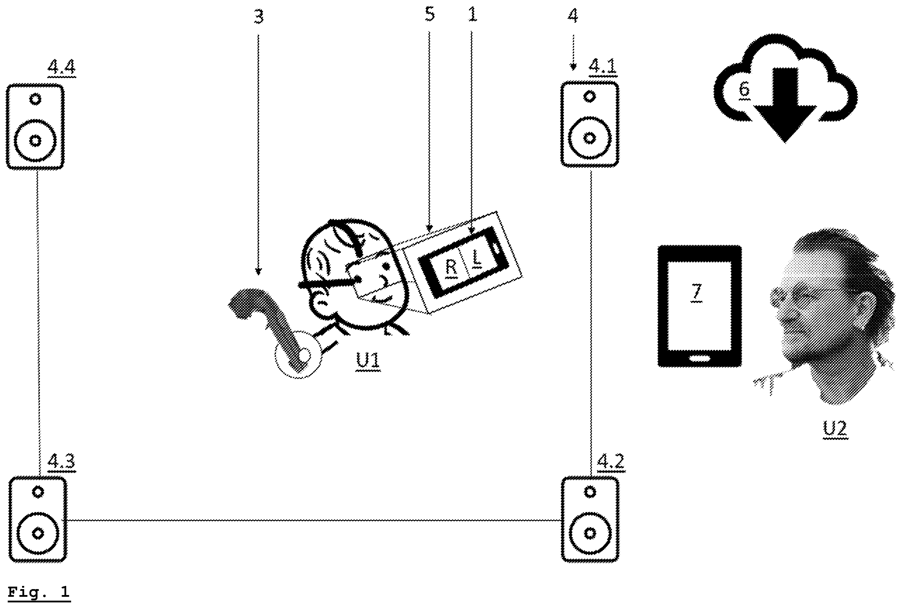

Fig. 1 shows a schematic view of the system according to the invention.

In Figure 1 a system 100 is shown for the treatment of amblyopia in a first user U1. The system consists of a computer 1 which is provided as a smartphone that is assembled with a head mount 5. The phone is provided with a software application, that is to say, the phone is programmed to generate a virtual environment. The phone produces a left eye image stream L and right eye image stream R of said environment adapted to be viewed dichoptically as a pair and perceived with binocular vision. In the example the phone's screen is provided as a dichoptic display (2). This is accomplished by the phone providing both streams in split screen while being assembled with the head mount 5 such that the image stream L and R reach are provided to the left eye and right eye respectively. In this example the streams L and R have a difference in information content, for example a different signal to noise ratio. This would also be true for laminated and partially obscured views in whatever design. The system 100 can further be seen to have a sound system 4. This sounds system consists of a plurality of speakers 4.1, 4.2, 4.3, 4.4 placed around the user so as to provide an immersive experience. The sound system may also separately from this example be wirelessly connected with the computer 1. This setup also ensures that the left ear and right ear of the user receive sounds as differently if produced at this source or these sources. This setup ensures that same sounds from one direction arrive at ever so slightly different volume and at different moments. This gives a sense of direction to the sound, which source the first user U1 may place at a certain location with respect to him or herself. The system 100 also comprises a hand held controller 3 which the first user may manipulate to perform tasks. The controller can furthermore be a wireless controller connecting to the computer via Bluetooth, although a wired connection may be possible. The phone may make use of its orientation sensor (not shown, but customary) to adjust the sounds from the sound system 4. The application on the phone gives access to a distant or local server 6 which is provided with a plurality of programs corresponding to mutually different virtual environments. Optionally, most of the virtual environment generation occurs server-side, although, the computer always outputs the environment to the first user, so in that sense, the computer also generates the environment for the user even though most calculations are performed server-side in such a case. Here the computer 1 communicates performance information to the server 6 for storage. Lastly, a second user U2 can be seen who monitors the progress of the first user via a separate human interface 7, such as a computer, laptop or tablet. This interface 7 is wirelessly connected to the server 6 for viewing performance of the first user U1. Optionally, also separately from this example the second interface allows the second user to change the environment or the change the difference in information content between streams L and R.

It should be understood that the tools and methods described herein above may alternatively be used for non-medical treatment purposes, such as to increase situational awareness and/or visual acuity, in otherwise healthy users. In one such example the system could aid in training first responders or emergency service personnel to have improved perceptive capabilities in chaotic environments.

## Claims

1. A system (100) for implementing a method of treatment of amblyopia, comprising:
- a computer (1) programmed to generate a virtual environment with a first user associated task reflective of binocular perception, wherein the computer is further designed to produce a left eye image stream and a right eye image stream of said environment adapted to be viewed dichoptically as a pair and perceived with binocular vision;
- a dichoptic display (2) designed to present a selected one of said image streams as a right eye image stream to a first user's right eye and a left eye image stream to a first user's left eye; and
- a sound system (4) comprising a plurality of sound sources (4.1, 4.2, 4.3, 4.4);
wherein the computer is programmed to manifest within said virtual environment at least one visual object in a first direction (D1) relative to the first user (U1), such as outside the field of vision of the user within the virtual environment, and
wherein the computer is programmed to produce sound streams of said environment, namely a continuous three dimensional soundscape comprising both environmental sounds and sounds pertaining to the manifested object, via the sound system (4) for directing the visual attention of the first user in said first direction (D1) within the virtual environment and wherein the visual streams have a mutual difference in information content between said left eye image stream and said right eye image
stream, wherein the system is designed such that the implementation of the method of treatment is based on measured performance related to tasks associated with the manifested object and excludes the tracking of eyes.

2. The system according to claim 1, wherein the sound streams of said environment are produced, such that, in use, auditory perception of the visual object by the first user precedes or coincides, such as exclusively precedes or coincides, with a visual perception of the visual object by the first user.

3. The system according to claim 1 or 2, wherein the sound stream comprises a left ear sound stream and a right ear sound stream, and wherein the streams comprise frequency, timing and/or loudness differences.

4. The system according to claim 1, 2 or 3, comprising a controller (3) for allowing a first user's input to perform the associated task.

5. The system according to claim 4, wherein the difference in information content is variable between said left eye image stream and said right eye image stream, and wherein the system comprises:
wherein the computer is optionally programmed to change the variable difference in information content between said left eye image stream and said right eye image stream in response to the first user's performance.

6. The system according to any one of claims 1-5, comprising a virtual reality head mount (5) to be worn by the first user, and wherein the computer (1) is designed as a smartphone assembled with said head mount, wherein the dichoptic display (2) is a screen comprised in the smartphone.

7. The system according to any one of claims 1-6, comprising a distant server (6), wherein the computer (1) is designed to retrieve the virtual environment and associated task from said server.

8. The system according to claim 7, wherein the system is designed such that the generated environment is provided as a selectable program from a digital library of programs for generating mutually different environments.

9. The system according to claim 8, designed to change the virtual environment to that of a different program from the digital library in response to the first user's performance.

10. The system according to claim 9, wherein the system records task performance information of the first user,
such as in the form of task completion, speed of completion and date of completion,
and wherein the system comprises a separate human interface for a second user, and wherein the system is designed to allow a second user to access task performance information of the first user via said separate human interface.

11. The system according to claim 10, wherein the second human interface enables the second user to select the program from the library.

12. The system according to any one of claims 1-11, wherein the sound system is designed to provide 360 degree spatial sound, such as by headphones, earbuds, or a headset, for allowing audio perception by the first user of the visual object to occur outside of a field of vision of the first user.

13. The system according to claim 12, comprising an orientation sensor, such as a 3D G-sensor or 3D magnetic field sensor, wherein the computer is designed to determine a change in direction of the first user's head using said sensor and wherein the computer is further designed to adjust the relative direction of the sound in response to the determined change in direction of the first user's head.

14. The system according to any one of claims 1-13, wherein the controller is communicatively connected to the computer, such as wirelessly.

15. A computer-implemented method for non-medical treatment comprising the step of:
- generating a virtual environment with a task reflective of binocular perception;
- producing a left eye image stream and right eye image stream of said environment adapted to be viewed dichoptically as a pair and perceived with binocular vision;
- presenting a selected one of said image streams as a right eye image stream and a left eye image stream; and
- manifesting an event with said virtual environment;-emitting sound streams corresponding to a direction or location of said event within the virtual environment;-measuring an input in response to the event,
wherein based on a measured performance related to tasks associated with a manifested object the method excludes the tracking of eyes.

16. A computer-implemented method according to claim 15, comprising the further steps of:
- changing the variable difference in information content between said left eye image stream and said right eye image stream in response to the received input.

17. A system for the treatment of amblyopia comprising:
- a source of binocular images having a difference in information content between left and right eye images ; and
- a source of sounds, corresponding to a direction and/or location of an object displayed in the binocular images with respect to a user,
- a user input device for receiving a user's input,
- a computer
programmed for generating the images and sounds, namely a first virtual gaming environment, and
programmed to assess a user's visual capacity by the user's input in tasks associated with the images, and
wherein the system is designed by the computer being programmed, to exclude the tracking of eyes, and wherein the computer is programmed to gradually adjust the difference in information content, in response to the assessment of the user's input so as to gradually improve the user's vision,
wherein the adjustment preferably comprises changing between mutually different gaming environments selected from a digital library, and wherein further preferably the visual requirements are increased for each subsequent environment, for example unless the user fails to navigate an environment.

## Patentansprüche

1. System (100) zum Implementieren eines Verfahrens zur Behandlung von Amblyopie, umfassend:
- einen Computer (1), der dazu programmiert ist, eine virtuelle Umgebung mit einer einem ersten Benutzer zugeordneten Aufgabe zu erzeugen, die die binokulare Wahrnehmung widerspiegelt, wobei der Computer ferner dazu ausgelegt ist, einen Linksaugen-Bildstrom und einen Rechtsaugen-Bildstrom der genannten Umgebung zu erzeugen, die dazu ausgelegt sind, dichoptisch als ein Paar betrachtet und mit binokularem Sehen wahrgenommen zu werden;
- eine dichoptische Anzeige (2), die dazu ausgelegt ist, einen ausgewählten der Bildströme als einen Rechtsaugen-Bildstrom für das rechte Auge eines ersten Benutzers und einen Linksaugen-Bildstrom für das linke Auge eines ersten Benutzers zu präsentieren; und
- ein Tonsystem (4) umfassend eine Vielzahl von Tonquellen (4.1, 4.2, 4.3, 4.4);
wobei der Computer dazu programmiert ist, innerhalb der virtuellen Umgebung mindestens ein visuelles Objekt in einer ersten Richtung (D1) relativ zu dem ersten Benutzer (U1) zu manifestieren, wie beispielsweise außerhalb des Sichtfeldes des Benutzers innerhalb der virtuellen Umgebung,
und
wobei der Computer dazu programmiert ist, über das Tonsystem (4) Klangströme der genannten Umgebung zu erzeugen, nämlich eine kontinuierliche dreidimensionale Klanglandschaft, die sowohl Umgebungsgeräusche als auch Geräusche umfasst, die zu dem manifestierten Objekt gehören, um die visuelle Aufmerksamkeit des ersten Benutzers in die genannte erste Richtung (D1) innerhalb der virtuellen Umgebung zu lenken, und
wobei die visuellen Ströme einen gegenseitigen Unterschied im Informationsgehalt zwischen dem Linksaugen-Bildstrom und dem Rechtsaugen-Bildstrom aufweisen, wobei das System so ausgelegt ist, dass das Implementieren des Behandlungsverfahrens auf einer gemessenen Leistung basiert, die sich auf Aufgaben bezieht, die mit dem manifestierten Objekt verbunden sind, und das Nachverfolgen der Augen ausschließt.

2. System nach Anspruch 1, wobei die Klangströme der genannten Umgebung derart erzeugt werden, dass im Gebrauch die auditive Wahrnehmung des visuellen Objekts durch den ersten Benutzer einer visuellen Wahrnehmung des visuellen Objekts durch den ersten Benutzer vorausgeht oder mit dieser zusammenfällt, wie beispielsweise ausschließlich vorausgeht oder zusammenfällt.

3. System nach Anspruch 1 oder 2, wobei der Klangstrom einen linken Ohrklangstrom und einen rechten Ohrklangstrom umfasst, und wobei die Ströme Frequenz-, Timing- und/oder Lautstärkeunterschiede aufweisen.

4. System nach Anspruch 1, 2 oder 3, umfassend einen Controller (3), um die Eingabe eines ersten Benutzers zum Durchführen der zugehörigen Aufgabe zu ermöglichen.

5. System nach Anspruch 4, wobei der Unterschied im Informationsgehalt zwischen dem Linksaugen-Bildstrom und dem Rechtsaugen-Bildstrom variabel ist, und wobei das System umfasst:
wobei der Computer wahlweise dazu programmiert ist, den variablen Unterschied im Informationsgehalt zwischen dem genannten Linksaugen-Bildstrom und dem genannten Rechtsaugen-Bildstrom in Reaktion auf die Leistung des ersten Benutzers zu verändern.

6. System nach einem der Ansprüche 1 bis 5, umfassend eine Virtual-Reality-Kopfhalterung (5), die vom ersten Benutzer zu tragen ist, und wobei der Computer (1) als ein mit der genannten Kopfhalterung zusammengesetztes Smartphone ausgebildet ist, wobei die dichoptische Anzeige (2) ein in dem Smartphone enthaltener Bildschirm ist.

7. System nach einem der Ansprüche 1 bis 6, umfassend einen entfernten Server (6), wobei der Computer (1) dazu ausgelegt ist, die virtuelle Umgebung und die zugehörige Aufgabe von dem Server abzurufen.

8. System nach Anspruch 7, **dadurch gekennzeichnet, dass** das System derart ausgelegt ist, dass die erzeugte Umgebung als ein auswählbares Programm aus einer digitalen Bibliothek von Programmen zum Erzeugen von gegenseitig unterschiedlichen Umgebungen bereitgestellt wird.

9. System nach Anspruch 8, das dazu ausgelegt ist, als Reaktion auf die Leistung des ersten Benutzers die virtuelle Umgebung in diejenige eines anderen Programms aus der digitalen Bibliothek zu verändern.

10. System nach Anspruch 9, wobei das System Aufgabenleistungsinformationen des ersten Benutzers aufzeichnet, beispielsweise in Form von Vollendung der Aufgabe, Geschwindigkeit der Vollendung und Datum der Vollendung,
und wobei das System eine separate menschliche Schnittstelle für einen zweiten Benutzer umfasst, und wobei das System dazu ausgelegt ist, einem zweiten Benutzer den Zugriff auf Aufgabenleistungsinformationen des ersten Benutzers über die separate menschliche Schnittstelle zu ermöglichen.

11. System nach Anspruch 10, wobei die zweite menschliche Schnittstelle den zweiten Benutzer in die Lage versetzt, das Programm aus der Bibliothek auszuwählen.

12. System nach einem der Ansprüche 1 bis 11, wobei das Tonsystem dazu ausgelegt ist, einen räumlichen 360-Grad-Klang bereitzustellen, beispielsweise durch Kopfhörer, Ohrstöpsel oder ein Headset, um die Audiowahrnehmung des visuellen Objekts durch den ersten Benutzer außerhalb des Sichtfelds des ersten Benutzers zu ermöglichen.

13. System nach Anspruch 12, umfassend einen Orientierungssensor, wie beispielsweise einen 3D-G-Sensor oder 3D-Magnetfeldsensor, wobei der Computer dazu ausgelegt ist, mithilfe des Sensors eine Richtungsänderung des Kopfes des ersten Benutzers zu bestimmen, und wobei der Computer ferner dazu ausgelegt ist, die relative Richtung des Klangs in Reaktion auf die bestimmte Richtungsänderung des Kopfes des ersten Benutzers anzupassen.

14. System nach einem der Ansprüche 1 bis 13, wobei der Controller kommunikativ mit dem Computer verbunden ist, wie beispielsweise drahtlos.

15. Computerimplementiertes Verfahren zur nicht-medizinischen Behandlung, umfassend den folgenden Schritt:
- Erzeugen einer virtuellen Umgebung mit einer Aufgabe, die die binokulare Wahrnehmung widerspiegelt;
- Erzeugen eines Linksaugen-Bildstroms und eines Rechtsaugen-Bildstroms der genannten Umgebung, die dazu angepasst sind, dichoptisch als Paar betrachtet und mit binokularem Sehen wahrgenommen zu werden;
- Präsentieren eines ausgewählten der genannten Bildströme als Rechtsaugen-Bildstrom und Linksaugen-Bildstrom; und
- Manifestieren eines Ereignisses in der genannten virtuellen Umgebung;
- Aussenden von Klangströmen, entsprechend einer Richtung oder einem Ort des genannten Ereignisses innerhalb der virtuellen Umgebung;
- Messen einer Eingabe als Reaktion auf das Ereignis, wobei, basierend auf einer gemessenen Leistung in Bezug auf Aufgaben, die mit einem manifestierten Objekt verbunden sind, das Verfahren das Nachverfolgen von Augen ausschließt.

16. Computerimplementiertes Verfahren nach Anspruch 15, umfassend die weiteren Schritte:
- Verändern des variablen Unterschieds im Informationsgehalt zwischen dem genannten Linksaugen-Bildstrom und dem genannten Rechtsaugen-Bildstrom in Reaktion auf die empfangene Eingabe.

17. System zur Behandlung von Amblyopie, umfassend:
- eine Quelle binokularer Bilder, die einen Unterschied im Informationsgehalt zwischen den linken und rechten Augenbildern aufweisen; und
- eine Klangquelle, die einer Richtung und/oder einem Ort eines auf den binokularen Bildern angezeigten Objekts in Bezug auf einen Benutzer entspricht,
- eine Benutzereingabevorrichtung zum Empfang der Benutzereingabe,
- einen Computer
programmiert, um die Bilder und Klänge zu erzeugen, nämlich eine erste virtuelle Spielumgebung, und
programmiert ist, um die visuelle Fähigkeit eines Benutzers anhand der Eingaben des Benutzers bei den mit den Bildern verbundenen Aufgaben zu bewerten, und wobei das System von dem zu programmierenden Computer dazu ausgelegt ist, das Verfolgen von Augen auszuschließen, und
wobei der Computer dazu programmiert ist, den Unterschied im Informationsgehalt als Reaktion auf das Bewerten der Eingaben des Benutzers allmählich anzupassen, um das Sehvermögen des Benutzers allmählich zu verbessern,
wobei das Anpassen vorzugsweise das Wechseln zwischen gegenseitig unterschiedlichen Spielumgebungen umfasst, die aus einer digitalen Bibliothek ausgewählt werden, und wobei ferner vorzugsweise die visuellen Anforderungen für jede nachfolgende Umgebung erhöht werden, zum Beispiel wenn es dem Benutzer nicht gelingt, durch eine Umgebung zu navigieren.

## Revendications

1. Système (100) pour la mise en œuvre d'un procédé de traitement de l'amblyopie, comprenant :
- un ordinateur (1) programmé pour générer un environnement virtuel comportant une première tâche utilisateur associée reflétant la perception binoculaire, l'ordinateur étant en outre conçu pour produire un flux d'images pour l'œil gauche et un flux d'images pour l'œil droit dudit environnement, adaptés pour être visualisés dichoptiquement en paire et perçus avec une vision binoculaire ;
- un dispositif dichoptique (2) conçu pour présenter l'un desdits flux d'images comme flux d'images pour l'œil droit à l'œil droit d'un premier utilisateur et l'autre flux d'images comme flux d'images pour l'œil gauche à l'œil gauche du premier utilisateur ; et
- un système sonore (4) comprenant une pluralité de sources sonores (4.1, 4.2, 4.3, 4.4) ;
dans lequel l'ordinateur est programmé pour manifester, dans ledit environnement virtuel, au moins un objet visuel dans une première direction (D1) par rapport au premier utilisateur (U1), par exemple en dehors du champ de vision de l'utilisateur dans l'environnement virtuel, et
dans lequel l'ordinateur est programmé pour produire des flux sonores dudit environnement, à savoir un paysage sonore tridimensionnel continu comprenant à la fois des sons environnementaux et des sons relatifs à l'objet manifesté, via le système sonore (4), afin de diriger l'attention visuelle du premier utilisateur dans ladite première direction (D1) dans l'environnement virtuel, et
dans lequel les flux visuels présentent une différence mutuelle de contenu d'information entre ledit flux d'images pour l'œil gauche et ledit flux d'images pour l'œil droit, le système étant conçu de sorte que la mise en œuvre du procédé de traitement est fondée sur une performance mesurée relative à des tâches associées à l'objet manifesté et exclut le suivi des yeux.

2. Système selon la revendication 1, dans lequel les flux sonores dudit environnement sont produits de telle sorte que, en utilisation, la perception auditive de l'objet visuel par le premier utilisateur précède ou coïncide, par exemple exclusivement précède ou coïncide, avec une perception visuelle de l'objet visuel par le premier utilisateur.

3. Système selon l'une quelconque des revendications 1 ou 2, dans lequel le flux sonore comprend un flux sonore pour l'oreille gauche et un flux sonore pour l'oreille droite, et dans lequel les flux présentent des différences de fréquence, de synchronisation et/ou de volume.

4. Système selon l'une quelconque des revendications 1 à 3, comprenant un contrôleur (3) permettant une entrée du premier utilisateur pour exécuter la tâche associée.

5. Système selon la revendication 4, dans lequel la différence de contenu d'information est variable entre ledit flux d'images pour l'œil gauche et ledit flux d'images pour l'œil droit, et dans lequel le système comprend :
- l'ordinateur étant éventuellement programmé pour modifier ladite différence variable de contenu d'information entre ledit flux d'images pour l'œil gauche et ledit flux d'images pour l'œil droit en réponse à la performance du premier utilisateur.

6. Système selon l'une quelconque des revendications 1 à 5, comprenant un casque de réalité virtuelle (5) destiné à être porté par le premier utilisateur, et dans lequel l'ordinateur (1) est conçu comme un smartphone assemblé audit casque, le dispositif dichoptique (2) étant un écran compris dans le smartphone.

7. Système selon l'une quelconque des revendications 1 à 6, comprenant un serveur distant (6), dans lequel l'ordinateur (1) est conçu pour récupérer l'environnement virtuel et la tâche associée depuis ledit serveur.

8. Système selon la revendication 7, dans lequel le système est conçu de sorte que l'environnement généré est fourni comme un programme sélectionnable à partir d'une bibliothèque numérique de programmes permettant de générer des environnements mutuellement différents.

9. Système selon la revendication 8, conçu pour modifier l'environnement virtuel vers celui d'un programme différent de la bibliothèque numérique en réponse à la performance du premier utilisateur.

10. Système selon la revendication 9, dans lequel le système enregistre des informations de performance du premier utilisateur relatives à la tâche, par exemple sous forme d'accomplissement de la tâche, de vitesse d'exécution et de date d'exécution,
et dans lequel le système comprend une interface humaine distincte pour un second utilisateur, le système étant conçu pour permettre au second utilisateur d'accéder aux informations de performance du premier utilisateur via ladite interface humaine distincte.

11. Système selon la revendication 10, dans lequel la seconde interface humaine permet au second utilisateur de sélectionner le programme dans la bibliothèque.

12. Système selon l'une quelconque des revendications 1 à 11, dans lequel le système sonore est conçu pour fournir un son spatial à 360 degrés, par exemple au moyen d'écouteurs, d'oreillettes ou d'un casque, afin de permettre une perception auditive par le premier utilisateur de l'objet visuel en dehors du champ de vision du premier utilisateur.

13. Système selon la revendication 12, comprenant un capteur d'orientation, tel qu'un capteur-G 3D ou un capteur de champ magnétique 3D, dans lequel l'ordinateur est conçu pour déterminer un changement de direction de la tête du premier utilisateur à l'aide dudit capteur et pour ajuster la direction relative du son en réponse au changement déterminé.

14. Système selon l'une quelconque des revendications 1 à 13, dans lequel le contrôleur est connecté de manière communicante à l'ordinateur, par exemple sans fil.

15. Procédé mis en œuvre par ordinateur pour un traitement non médical, comprenant les étapes consistant à :
- générer un environnement virtuel avec une tâche reflétant la perception binoculaire ;
- produire un flux d'images pour l'œil gauche et un flux d'images pour l'œil droit dudit environnement, adaptés pour être visualisés dichoptiquement en paire et perçus avec une vision binoculaire ;
- présenter l'un desdits flux d'images comme flux d'images pour l'œil droit et l'autre comme flux d'images pour l'œil gauche ; et
- manifester un événement dans ledit environnement virtuel ;
- émettre des flux sonores correspondant à une direction ou une localisation dudit événement dans l'environnement virtuel ;
- mesurer une entrée en réponse à l'événement,
dans lequel, sur la base d'une performance mesurée relative à des tâches associées à un objet manifesté, le procédé exclut le suivi des yeux.

16. Procédé selon la revendication 15, comprenant en outre l'étape consistant à modifier la différence variable de contenu d'information entre ledit flux d'images pour l'œil gauche et ledit flux d'images pour l'œil droit en réponse à l'entrée reçue.

17. Système pour le traitement de l'amblyopie comprenant :
- une source d'images binoculaires présentant une différence de contenu d'information entre les images pour l'œil gauche et pour l'œil droit ; et
- une source de sons correspondant à une direction et/ou une localisation d'un objet affiché dans les images binoculaires par rapport à un utilisateur,
- un dispositif d'entrée utilisateur pour recevoir une entrée de l'utilisateur,
- un ordinateur programmé pour générer les images et les sons, à savoir un premier environnement de jeu virtuel, et programmé pour évaluer la capacité visuelle d'un utilisateur par l'entrée de l'utilisateur dans des tâches associées aux images, et
dans lequel le système est conçu, par programmation de l'ordinateur, pour exclure le suivi des yeux, et
dans lequel l'ordinateur est programmé pour ajuster progressivement la différence de contenu d'information en réponse à l'évaluation de l'entrée de l'utilisateur de manière à améliorer progressivement la vision de l'utilisateur, l'ajustement comprenant de préférence un changement entre des environnements de jeu mutuellement différents sélectionnés à partir d'une bibliothèque numérique, et dans lequel, de préférence, les exigences visuelles sont augmentées pour chaque environnement subséquent, par exemple sauf si l'utilisateur ne parvient pas à naviguer dans un environnement.
